(19) Europäisches Patentamt
European Patent Office
Office européen des brevets

(11) **EP 3 245 953 B1**

(12) **EUROPEAN PATENT SPECIFICATION**

(45) Date of publication and mention
of the grant of the patent:
**24.04.2019 Bulletin 2019/17**

(51) Int Cl.:
***A61B 8/00*** *(2006.01)*

(21) Application number: **17169270.0**

(22) Date of filing: **03.05.2017**

(54) **SYSTEM FOR SENSING POSITION OF IMAGING DEVICE**

SYSTEM ZUR ERFASSUNG DER POSITION EINER ABBILDUNGSVORRICHTUNG

SYSTÈME DE DÉTECTION DE POSITION DE DISPOSITIF D'IMAGERIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.05.2016 US 201615146329**

(43) Date of publication of application:
**22.11.2017 Bulletin 2017/47**

(73) Proprietor: **Covidien LP
Mansfield, MA 02048 (US)**

(72) Inventors:
• **HILL, Morgan L.
Boulder, Colorado 80301 (US)**
• **LARSON, Eric W
Littleton, Colorado 80127 (US)**
• **MANKAR, Nikhil
411057 Pune (IN)**
• **RESCHKE, Arlen
Longmont, Colorado 80501 (US)**
• **BHARADWAJ, Jeetendra
Lafayette, Colorado 80026 (US)**

(74) Representative: **Maschio & Soames IP Ltd
30 Carlton Crescent
Southampton SO15 2EW (GB)**

(56) References cited:
**EP-A1- 1 932 477       JP-A- 2013 141 575
US-A1- 2009 266 957     US-A1- 2010 081 920
US-A1- 2014 243 671     US-A1- 2015 105 662**

## Description

## BACKGROUND

### 1. Technical Field

The present disclosure relates to systems and devices for sensing a position of an imaging device. More particularly, the present disclosure relates to sensor mounts for an imaging device of a treatment system during performance of a treatment procedure.

### 2. Discussion of Related Art

When treating patients, clinicians often rely on patient data including X-ray data, computed tomography (CT) scan data, magnetic resonance imaging (MRI) data, or other imaging data that allows the clinician to view the internal anatomy of a patient. The clinician utilizes the patient data to identify targets of interest and to develop strategies for accessing the targets of interest for the surgical procedure.

This off-line information, however, does not enable a clinician to effectively track a location of a treatment probe through and inside of a patient's body and perform a surgical procedure. Further, relative positional information between the treatment probe and the targets cannot be retrieved from the off-line information during surgical operations. Thus, real-time images are needed to facilitate the surgical procedure and an imaging device is used to image the treatment probe and surrounding tissues during surgical operations.

US 2010/0081920 A1 can be considered as disclosing the closest prior art for the invention. It discloses a system for sensing the position of an ultrasound imaging device comprising

- an ultrasound imaging device,
- a position sensor mount including an elastic body portion, and
- a position sensor mounted on the position sensor mount and configured to sense position information of the ultrasound imaging device in a coordinate system;

wherein the ultrasound imaging device is received by the position sensor mount. The elastic body portion is formed by a ring-like body member arranged to snap-fit about a portion of the periphery of the probe. The position sensor is mounted in a socket formed by an upstanding portion of the position sensor mount.

## SUMMARY

The present disclosure is directed to systems and devices for sensing a position of an imaging device of a treatment system during surgical procedure are provided herein below.

In one aspect of the present disclosure there is described a system for sensing a position of an ultrasound imaging device includes an ultrasound (US) imaging device, and a position sensor mount, and a position sensor. The position sensor mount includes an elastic body portion, a first rigid shell disposed on a distal portion of the elastic body portion, a second rigid shell disposed on a proximal portion of the elastic body portion, and a first adhesive disposed on the first rigid shell. The position sensor is mounted on the first rigid shell and configured to sense position information of the US imaging device in a coordinate system. The US imaging device is received by the position sensor mount and circumscribed by the first adhesive.

In a further aspect, the first rigid shell includes a first recess and a second recess, which have a same angle with respect to a longitudinal axis of the US imaging device in an opposite direction. The position sensor includes a first sensor and a second sensor, which are 5 degrees of freedom sensors. The first sensor is positioned on the first recess and the second sensor is positioned on the second recess.

In another aspect, the position sensor includes a conductor configured to transmit sensed result. The elastic body portion includes a recess along a longitudinal axis, and the conductor is positioned on the recess of the elastic body portion. The second rigid shell includes a recess along the longitudinal axis of the US imaging device, and the conductor is positioned on the recess of the second rigid shell.

In still another aspect, the coordinate system is based on an electromagnetic field, and the position sensor is an electromagnetic sensor. The position sensor may be located at a first predetermined distance from a distal tip of the US imaging device and the position information of the US imaging device is based on sensed results of the position sensor and the first predetermined distance. Further, the US imaging device may include a notch having an angle along a longitudinal axis of the US imaging device. The system further includes a second adhesive formed on the first rigid shell circumscribes the notch following the angle.

## BRIEF DESCRIPTION OF THE DRAWINGS

Objects and features of the presently disclosed systems and devices will become apparent to those of ordinary skill in the art when descriptions of various embodiments thereof are read with reference to the accompanying drawings, of which:

FIG. 1 is a schematic diagram of a tracking and treatment system in accordance with an illustrative embodiment of the present disclosure;
FIG. 2A is a perspective view of the distal portion of an imaging device of the system of FIG. 1 in accordance with an embodiment of the present disclosure;
FIG. 2B is a side view of the distal portion of the imaging device of the FIG. 2A;

FIG. 3 is a perspective view of a sensor mount for the imaging device of FIG. 1 in accordance with an embodiment of the present disclosure;

FIGS. 4A-4E are illustrations of steps of a method for securely mounting a position sensor to the imaging device using the sensor mount in accordance with an embodiment of the present disclosure; and

FIG. 5 is a schematic diagram of a computing device 500 which forms part of the tracking and treatment system 10 of FIG. 1 in accordance with an embodiment of the present disclosure.

## DETAILED DESCRIPTION

The present disclosure provides a treatment system for tracking and imaging treatment probes, each of which includes a position sensor mounted thereon. While performing a surgical treatment, it is important to know exactly where a treatment probe is located within the patient's body with respect to a target for treatment. In addition, it is beneficial to see an actual image of the treatment probe as it is traversing tissue or entering the target. In this regard, the present disclosure describes a sensor mount configured to mount a position sensor on an imaging device. This enables the system to track relative spatial relationship between the treatment probe and the imaging device and to track the positions of both within the patient's body.

Position sensors are mounted on the imaging device to track the location of the imaging device and to provide an orientation of an imaging plane of the imaging device. The imaging plane of the imaging device is identified by a predetermined distance and orientation between the location of the position sensor and the location of the transducer of the imaging device. Thus, firm mounting of the position sensors is necessary to ensure that the correct location of the imaging device probe and the orientation imaging plane of the imaging device is identified and depicted on a display.

Further, when an imaging device is inserted inside a patient, the imaging device needs to bend or move in order to navigate a pathway in order to be positioned at the proper location to capture desired images of the treatment probe and the surrounding organs. In order to get accurate location information of the treatment probe with respect to the imaging device, a position sensor needs to be securely attached to the imaging device. Also, when the imaging device moves or the distal tip of the imaging device bends to a direction different from movements of the imaging device, the position sensor needs to be secured and not moved relative to the distal tip of the imaging device.

A system according to the present disclosure may be a unitary system configured to perform both the planning phase and the treatment phase, or the system may include separate devices and software programs for various phases. An example of the latter may be a system wherein a first computing device with one or more specialized software programs is used during the planning phase, and a second computing device with one or more specialized software programs may import data from the first computing device to be used during the treatment phase.

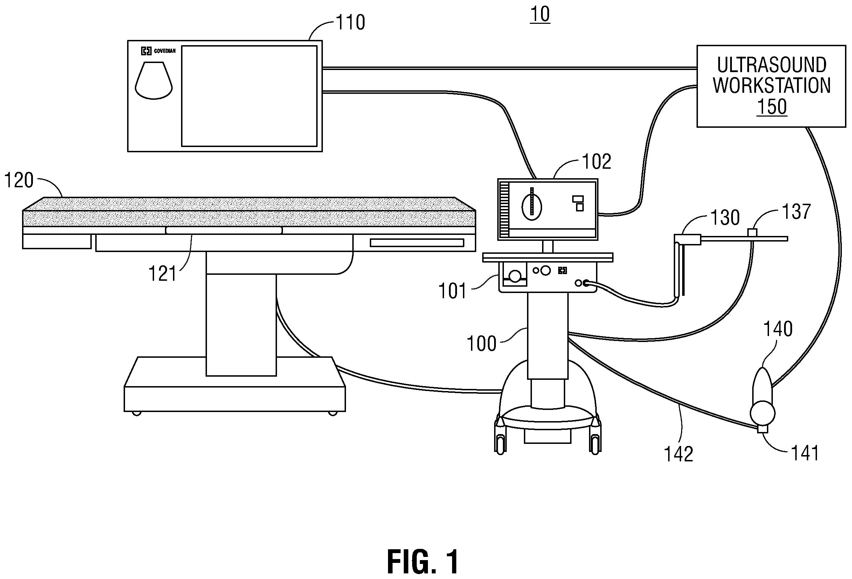

Referring now to FIG. 1, the present disclosure is generally directed to a tracking and treatment system 10, which includes an EM tracking workstation 100, an electrosurgical generator 101, a control workstation 102, a display 110, a table 120, a treatment probe 130, an imaging device 140, and an ultrasound workstation 150. As will be described in greater detail herein, the EM tracking workstation 100 may be, for example, a laptop computer, desktop computer, tablet computer, or other similar device which executes a special program to carry out the functions of the tracking system. The EM tracking workstation 100 includes software which converts signals received from the position sensors 137 and 141 and performs necessary calculations to track the location of the EM sensors in an EM field generated by the EM field generator 121. The EM tracking workstation 100 may interact with one or more position sensors 137 and 141 positioned on the treatment probe 130 positioned on the imaging device 140 (e.g., an EM sensor, though others could be used), respectively, and the display 110 on which a user interface presents an image of the location of the position sensors 137 in the EM field in combination with one or more imaging modalities. In addition to tracking information, the display 110 presents to a user the results of the software processing including instructions, images, and messages relating to the performance of the procedure.

The EM field generator 121 may rest on or may be built into the table 120 generate an EM field around a portion of the patient's body through which navigation to a target is desired. Alternatively the EM field generator 121 may simply be placed securely near the patient (e.g., using a boom or other support) and generate an EM field around all or a portion of the patient's body through which navigation to a target is desired. Typically, this may be the patient's torso which enables navigation to and treatment of all the major organs of the body. However, the same system could be used to treat other locations on the patient. An example of such an EM field generator is available under the brand name AURORA™ and sold by Northern Digital Inc.

The electrosurgical generator 101 generates electrosurgical energy (e.g., RF or microwave) and provides the generated energy to the treatment probe 130. The treatment probe 130 is a surgical instrument, for example, a microwave ablation antenna used to ablate and treat tissue. Various other surgical instruments or surgical tools, such as electrosurgical pencils, vessel sealers, staplers, resection devices and others, may also be used with EM tracking workstation 100 either with or without the position sensor 137. In one embodiment, the position sensor 137 located is on the treatment probe 130 as will be described in detail below, allowing for the tracking of the location of the treatment probe 130 in the EM field. While the present disclosure describes the use of the system 10 in a surgical environment, it is also envisioned that some or all of the components of system 10 may be used in alternative settings, for example, at a treatment review board or other office setting such as during a post treatment review of the procedure or assessment of progress of the patient.

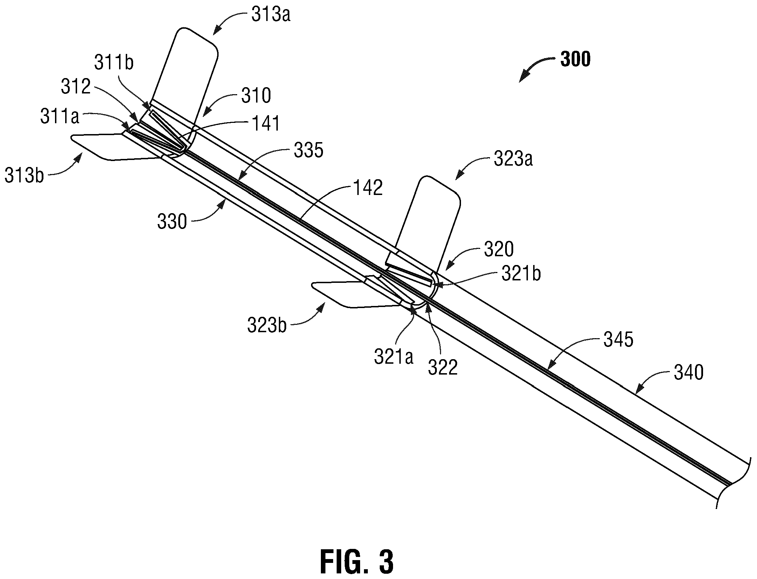

Along with the EM tracking workstation 100, the system 10 includes the capabilities for patient, target, and the treatment probe 130 visualization using ultrasonic imaging. The imaging device 140, such as an ultrasonic wand, may be used to image the patient's body during the procedure to visualize the location of the surgical instruments, such as the treatment probe 130, inside the patient's body. The imaging device 140 may also have an EM position sensor 141 mounted on the ultrasonic wand via a sensor mount 300 (shown in FIG. 3). As described further below, the imaging device 140 may be positioned in relation to the treatment probe 130 such that the treatment probe 130 is disposed at an angle to the ultrasound image plane, thereby enabling the clinician to visualize the spatial relationship of the treatment probe 130 with the ultrasound image plane and with objects being imaged. Further, the EM tracking workstation 100 may also track the location of imaging device 140 using the position sensor 141 placed thereon.

The position sensors 141 and 137 may be a six degrees of freedom (DOF) sensor or may be implemented as two five DOF sensors. Examples of such sensors include the AURORA® 5 DOF sensor and the AURORA® mini or micro 6 DOF sensors sold by Northern Digital Inc.

The position sensor 141, as a 6 DOF sensor, senses X-Y-Z location data as well as pitch, roll and yaw of the sensor as it is moved. When the position sensor 141 senses and transmits strengths or variations of the EM field in 6 directions via a sensor wire 142, the EM tracking workstation 100 is capable of calculating the location, translation, and rotation of the position sensor 141 with respect to the imaging device 140. Based on these rotational and translational movements, the ultrasound image plane moves and correspondingly on the display 110. 5 DOF sensors only provide X-Y-Z location data and two of pitch, roll or yaw, but not all three. However, through the use of two such sensors, the missing orientation data can be calculated. This can be particularly useful in scenarios where size is important, such as in laparoscopic surgery where tools are typically designed with a maximum trocar inner diameter as the size limit. As a result the addition of a single 6 DOF sensor might be impossible where it has a nominal diameter of 0.8 mm, in contrast a 5 DOF sensor can be as small as 0.5 mm.

The control workstation 102 may interact with and control the electrosurgical generator 101. The control workstation 102 may further combine the ultrasound images from the ultrasound workstation 150 and EM data from the EM tracking workstation 100. The EM data may include spatial relationship between the location of the imaging device 140 and the location of the treatment probe 130 in the EM field. Based on the spatial relationship, the control workstation 102 generates images depicting the location of the treatment probe 130 with respect to pre-stored images illustrating the treatment probe 130 on display 110. In addition the control workstation 102 generates a representation of the location of the treatment probe in relation to the ultrasound images such that the treatment probe 130 is depicted with respect to the imaging device 140 and any pre-planned pathway to a target in the ultrasound image is also displayed allowing the clinician to follow the pathway and achieve the target.

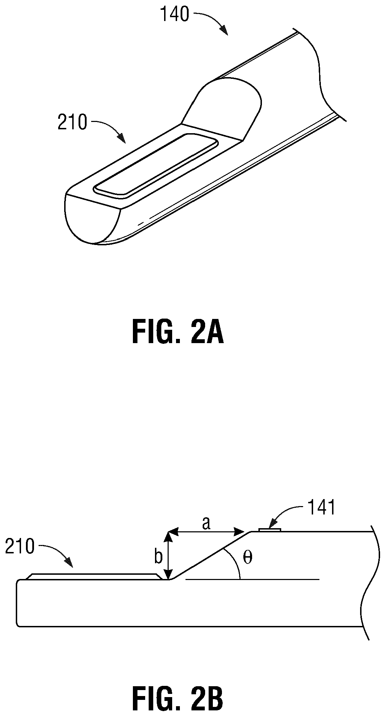

Now referring to FIGS. 2A and 2B, a structural profile of the distal portion of the imaging device 140 is shown in accordance with an embodiment of the present disclosure. For example FIGS. 2A and 2B may be the distal portion of a 4-way ultrasound probe where a transducer 210 is located. More particularly, as shown, the transducer 210 is located at a position distal to and vertically separated from the location of the position sensor 141. For example, the transducer is spaced apart from the position sensor 141 by a distance "a" longitudinally and a distance "b" vertically. As a result, the inclination between the transducer 210 and the position sensor 141 forms an angle $\theta$ with respect to the longitudinal axis of the imaging device 140. The EM tracking workstation 100 calculates the location of the distal tip of the imaging device 140 based on this structural profile or longitudinal and lateral distances "a" and "b." The predetermined distance between the proximal end of the transducer 210 and the position sensor 141 may be (a, b) in Cartesian coordinate form or $\left(\sqrt{a^2+b^2},\theta\right)$ in polar form. Utilizing this relationship, when the location of the imaging device 140 is detected in an electromagnetic field, an ultrasound scanning plane, and the location of the distal end of the imaging device 140 within the EM field can be calculated.

Turning now to FIG. 3, there is shown a sensor mount 300 for the imaging device 140 of FIG. 1. The sensor mount 300 mounts a position sensor 141 (the EM position sensor 141 shown in FIGS. 1 and 2B) to the imaging device 140. The imaging device 140 may need to bend in one or more directions which are different from the direction of movement thereof to capture desired images of the patient and the treatment probe 130 based on the location of the target or the location of the treatment probe 130. When the imaging device 140 bends or moves, the sensor mount 300 provides a securing means so that the position sensor 141 is securely attached to the imaging device 140 and does not move relative to the distal tip of the imaging device 140.

The sensor mount 300 may include three parts, first shell 310, second shell 320, and an elastic body portion 330 such as an elongated body portion. The first and second shells are formed to have an inner surface which contour matches that of the outer surface of the imaging device 140. Further, the rigidity of the first and second shells 310 and 320 provides firm securement of the position sensor 141 to the imaging device 140 as will be described in greater detail below. In an aspect, the first and second shells 310 and 320 may be deflectable and used as a snap-in to form a firm attachment with the imaging device 140. The elastic body portion 330 is formed to flex, for example, it may be made of any suitable elastic material that provides sufficient flexibility so that as the imaging device 140 bends or moves it can do so without the position sensor 141 moving relative to the distal tip of the imaging device 140.

The first shell 310 is firmly connected to the distal end of the elastic body portion 330 and the second shell 320 is also firmly connected to the proximal end of the elastic body portion 330. In an aspect, the first and second shells 310 and 320 may be separate from the elastic body portion 330 and may be assembled together when used. In this manner, elastic body portions of different lengths may be used depending on the desired separation distances for a given imaging device to which the sensor mount 300 is to be attached. Alternatively, one or both of the first and second shells 310 and 320 may be adapted to slide relative the elastic body portion 330, for example, by way of forming a sliding collar, so as to enable selective precise positioning for a particular instrument. Such an arrangement could be indexed with specific separation distances marked for user or instrument needs.

The first shell 310 may include a plurality of recesses for receiving the position sensor 141 and/or a sensor wire 142. The plurality of recesses may include a first sensor path 311a, a second sensor path 311b, and a sensor wire path 312.

The two 5 DOF sensors (e.g., the position sensor 141) may be placed on the first and second sensor paths 311a and 311b, respectively. The recesses of the first and second sensor paths 311a, 311b may secure the two 5 DOF sensors. The first and second sensor paths 311a, 311b may form the same angle with respect to the longitudinal axis of the imaging device 140 in opposite direction with each other. For example, the first sensor path 311a forms an angle, such as 10 or 15 degrees, from the longitudinal axis to its right and the second sensor path 311b forms the same angle from the longitudinal axis to its left. In an aspect, the recess may be filled with adhesive so that the first or second sensor is securely attached in the recess. In another aspect, adhesive may be applied to non-recess area so that, when the sensor mount 300 wholly or partially circumscribes the imaging device 140, the sensor mount 300 fixedly adheres to the imaging device 140 and the position sensor 141 cannot move relatively with respect to the distal end of the imaging device 140.

The sensor wire path 312 of the first shell 310 may extend along the longitudinal axis of the imaging device 140 in the form of a recess. The sensor wire path 312 is located in the middle between the first and second sensor paths 311a and 311b and may receive a sensor wire 142 of the position sensor 141 in a way similar to the first or second sensor path receives the position sensor including two sub-sensors, each of which is capable of sensing 5 DOF. In a case when the position sensor 141 has only one 6 DOF sensor, the position sensor 141 may be positioned in a distal portion of the sensor wire path 312, which is positioned between the first and second sensor paths 311a and 311b.

The first shell 310 may include an adhesive tab 313, which is an elongated tape material coated with an adhesive suitable for surrounding the imaging device 140 to securely attach the sensor mount 300 to the imaging device 140. The adhesive tab 313 may extend in a direction perpendicular to the longitudinal axis of the imaging device 140. The adhesive tab 313 may be formed to extend from the two opposite sides of the first shell 310 to form portions 313a and 313b so as to be able to partially or totally surround the imaging device 140 from both directions.

In an aspect, the adhesive tab 313 may include a releasable tab, which makes it easier to release or peel away the adhesive tab 313 from the imaging device 140 after a surgical operation. The releasable tab may protrude from the distal end of the adhesive tab 313. In another aspect, the adhesive tab 313 may include a recess at the distal end of the adhesive tab 313 so that the adhesive tab 313 can be easily released or peeled off. In this case, the distal portion of the adhesive tab 313 has a surface area sufficient to provide firm attachment to the imaging device 140.

The second shell 320 may be similar or identical to the first shell 310 and includes a first sensor path 321a, a second sensor path 321b, and a wire path 322. In the embodiment shown in FIG. 3, the first and second sensor paths 321a and 321b are not utilized. Instead the sensor wire simply traverses the wire path 322.

The second shell 320 also includes an adhesive tab 323, which surrounds the imaging device 140. Thus, the adhesive tab 313 and the adhesive tab 323 are used to firmly attach the sensor mount 300 to the imaging device 140 at the distal end and at the proximal end of the imaging device 140, respectively. The adhesive tab 323 may be formed to extend from the two opposite sides of the second shell 320 to form portions 323a and 323b so as to be able to partially or totally surround the imaging device 140 from both directions. These adhesive tabs 313, 323 prevent both axial and rotational movement of the sensor mount 300 with respect to the longitudinal axis of the imaging device 140. In this way, the position sensor 141, which is fixedly mounted on the sensor mount 300, maintains a predetermined distance from the transducer 210, and the EM tracking workstation 100 can determine the location, movement, and rotation of the imaging device 140.

The second shell 320 may extend toward the proximal end of the imaging device 140 along the longitudinal axis. In an aspect, the second shell 320 may have the same length as the first shell 310. In another aspect, the sensor mount 300 may further include a third shell 340, which is connected to the proximal portion of the second shell 320 or formed unitarily therewith. The third shell 340 may include one long recess (e.g., a sensor wire path) 345 along the longitudinal axis, which receives a sensor wire 142. In an aspect, adhesive may be applied to the sensor wire path 345 so that the sensor wire 142 can be fixedly secured in the sensor wire path 345. In another aspect, adhesive may be applied to areas other than the sensor wire path 345 so that the third shell 340 is securely attached to the imaging device 140.

The elastic body portion 330 is flexible to allow for flexure of the imaging device 140. As described above, the imaging device 140 may be a 4-way ultrasound probe and thus bend in four separate directions from the longitudinal axis of the imaging device and as much as 90 degrees in each direction. As the imaging device 140 bends, the length of the one side of the imaging device increases by the degree of bend, similarly the length on the opposite side (e.g., inside of the bend) decreases. As a result, in order to maintain the position of the first and second shells 310 and 320 the elastic backing material must extend to accommodate the movements. So, when the imaging device 140 bends, the elastic body portion 330 stretches without affecting the position of the mounted position sensor 141 with respect to the distal tip of the imaging device 140.

The elastic body portion 330 may include a recess 335 to receive a sensor wire or other means to hold the sensor wire, such as an adhesive, hook and loop fasteners, a retaining loop of materials similar to that of the elastic body portion 330 and others. Thus, when the position sensor(s) 141 is mounted on the first shell 310 of the sensor mount 300 the sensor wire extends along the elastic body portion 330 and through recess 335 and is placed over the second shell 320 and the elastic body portion 330, the position sensor 141 can be connected to the sensor wire.

In an aspect, the sensor mount 300 may be made of any suitable materials which do not pose any significant interference with the EM field generated by the EM field generator 121 and signals generated by the imaging device 140.

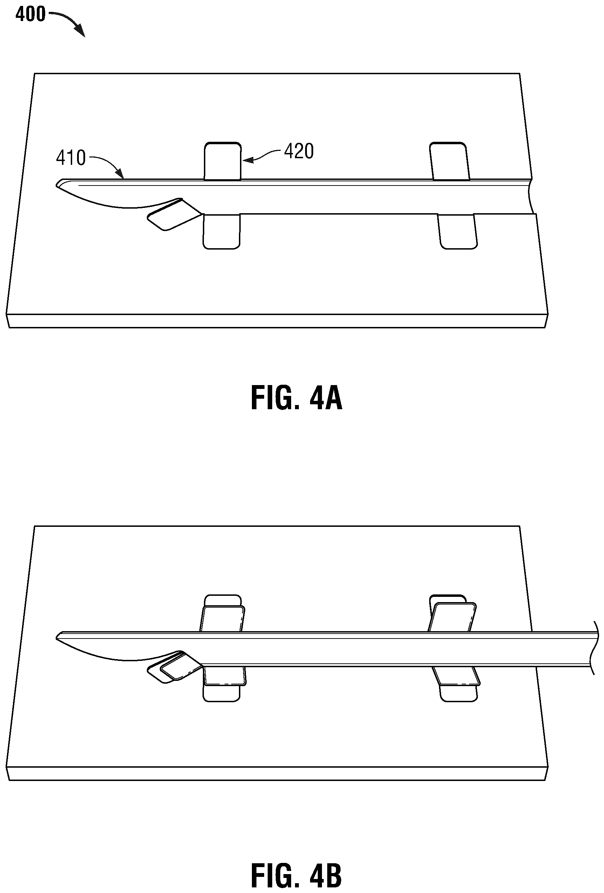

Even if the sensor mount 300 prevents the position sensor 141 from rotational and translational movements relative to imaging device 140 after it is attached to the device, it is also important to attach the sensor mount 300 at a proper location of the imaging device 140 to keep the predetermined distance between the position sensor 141 and the transducer 210 constant. FIGS. 4A-4E illustrate a method of attaching the sensor mount 300 to the imaging device 140 at a proper position.

FIG. 4A shows an assembly fixture 400 for the imaging device 140, which includes a first pattern 410 for the imaging device 140 and a second pattern 420 for the sensor mount 300. Assembly fixture 400 facilitates the precise attachment of sensor mount 300 to imaging device 140. The first pattern 410 corresponds to the structural profile of imaging device 140 and the second pattern 420 corresponds to the structural profile of the sensor mount 300 for mounting on the imaging device 140. In practice, the sensor mount 300 is laid in the second pattern 420 such that the adhesive tabs 313 and 323 are located in the adhesive tab channels. Once so placed, the imaging device 140 is placed into the assembly fixture 400 on top of the sensor mount 300 and over the first pattern 410. The main and adhesive tab channels ensure the proper precise and repeatable placement of the sensor mount 300 and the position sensor 141 on the imaging device 140, as shown in FIG. 4B. In an aspect, the first and second patterns 410, 420 may be manufactured by vacuum molding, injection molding, machining, engraving, etc.

In one embodiment, the sensor mount 300 may include an additional adhesive tab 314 connected at an angle to the first shell 310, as shown in FIG. 4B. The additional adhesive tab 314 is located at the inclination of the imaging device 140 and can provide greater security against movement of the position sensor 141 with respect to the imaging device 140. The additional adhesive tab 314 may form an angle with the first and second sensor paths 311a and 311b, which is substantially similar to the angle θ of the inclination, as described above with respect to the structural profile of the imaging device 140.

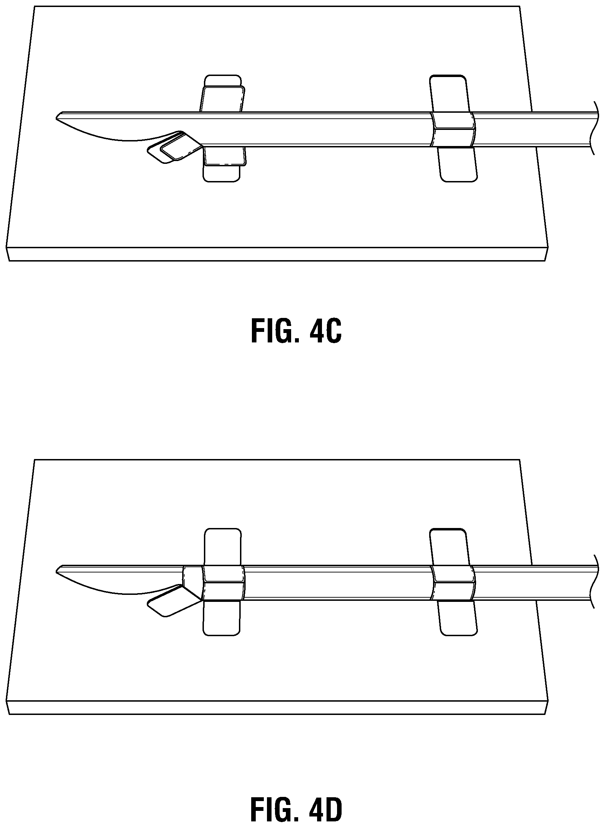

After the imaging device 140 is located over the pattern 410, the adhesive tabs 313a, 313b surround the imaging device 140 to fixedly attach the sensor mount 300 to the imaging device 140 as shown in FIG. 4C. In an aspect, the additional adhesive tab 314 is also used to surround the inclination of the imaging device 140, as shown in FIG. 4D. In this way, the sensor mount 300 is attached to the imaging device 140 at the desired location all the times and, as a result, the predetermined distance between the distal tip of the position sensor 141 and the proximal end of the transducer 210 is maintained constant.

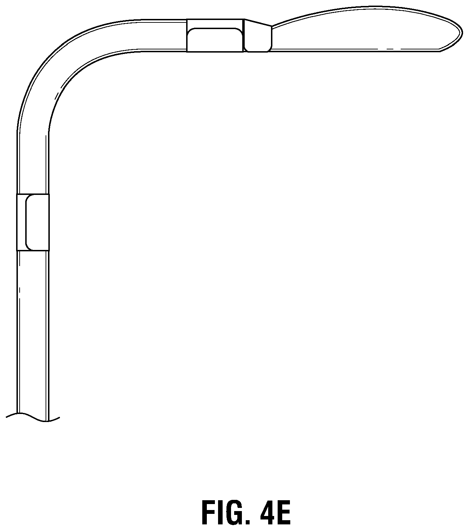

FIG. 4E illustrates bending of the imaging device 140. Since the first shell 310 moves with the transducer 210 and the elastic body portion 330 follows the bending, the predetermined distance between the position sensor 141 and the transducer 210 is also maintained during the bending. As can be appreciated the sensor wire 142 of the position sensor 141, must be allowed to slide through the wire path 322 of the second shell 320, and to some extent through recess 335 of the elastic body portion 330 as the four-way imaging device 140 bends. This movement is required as a result of the increase or decrease in distance between the first and second shells 310, 320 as the imaging device 140 bends. As shown in Fig. 4E the distance is increased as compared to Fig. 4B. Without such sliding the position sensor 141 might be pulled off of its position on the imaging device 140 as a result of this movement.

In an aspect, the imaging device 140 and the sensor mount 300 may be covered by a sterilized cover (e.g., a prophylactic) to prevent contamination, or infection in a patient while imaging.

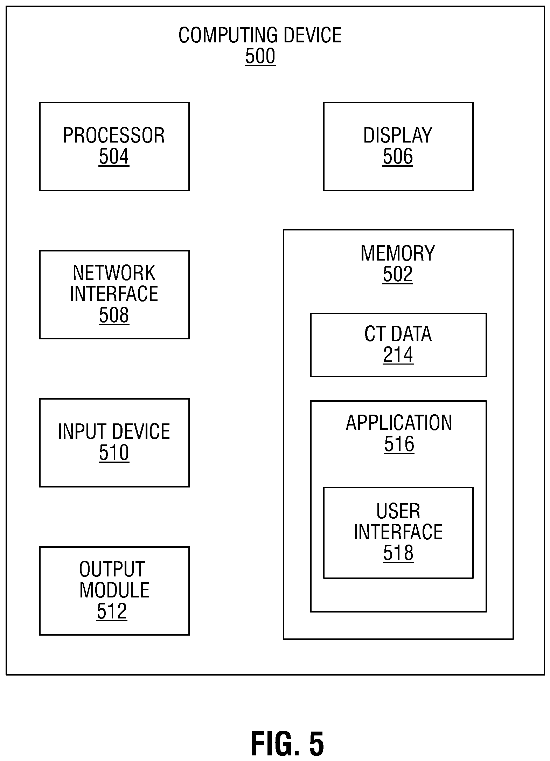

Turning now to FIG. 5, there is shown a system diagram of a computing device 500, which can be the EM tracking workstation 100, the control workstation 102, or the ultrasound workstation 150. The computing device 500 may include memory 502, processor 504, the display 506, network interface 508, input device 510, and/or output module 512.

Memory 502 includes any non-transitory computer-readable storage media for storing data and/or software that is executable by processor 504 and which controls the operation of the computing device 500. In an embodiment, memory 502 may include one or more solid-state storage devices such as flash memory chips. Alternatively or in addition to the one or more solid-state storage devices, memory 502 may include one or more mass storage devices connected to the processor 504 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 504. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by the computing device 500.

Memory 502 may store application 516 and/or CT data 514. Application 516 may, when executed by processor 504, cause the display 506 to present user interface 518.

Processor 504 may be a general purpose processor, a specialized graphics processing unit (GPU) configured to perform specific graphics processing tasks while freeing up the general purpose processor to perform other tasks, and/or any number or combination of such processors.

The display 506 may be touch-sensitive and/or voice-activated, enabling the display 506 to serve as both an input and output device. Alternatively, a keyboard (not shown), mouse (not shown), or other data input devices may be employed.

Network interface 508 may be configured to connect to a network such as a local area network (LAN) consisting of a wired network and/or a wireless network, a wide area network (WAN), a wireless mobile network, a Bluetooth network, and/or the internet. For example, the computing device 500 may receive computed tomographic (CT) image data of a patient from a server, for example, a hospital server, internet server, or other similar servers, for use during surgical ablation planning. Patient CT image data may also be provided to the computing device 500 via a removable memory 502. The computing device 500 may receive updates to its software, for example, application 516, via network interface 508. The computing device 500 may also display notifications on the display 506 that a software update is available.

Input device 510 may be any device by means of which a user may interact with the computing device 500, such as, for example, a mouse, keyboard, foot pedal, touch screen, and/or voice interface.

Output module 512 may include any connectivity port or bus, such as, for example, parallel ports, serial ports, universal serial busses (USB), or any other similar connectivity port known to those skilled in the art.

Application 516 may be one or more software programs stored in memory 502 and executed by processor 504 of the computing device 500. During a planning phase, application 516 guides a clinician through a series of steps to identify a target, size the target, size a treatment zone, and/or determine an access route to the target for later use during the procedure phase. In some embodiments, application 516 is loaded on computing devices in an operating room or other facility where surgical procedures are performed, and is used as a plan or map to guide a clinician performing a surgical procedure, but without any feedback from the treatment probe 130 used in the procedure to indicate where the treatment probe 130 is located in relation to the plan

Application 516 may be installed directly on the computing device 500, or may be installed on another computer, for example a central server, and opened on the computing device 500 via network interface 508. Application 516 may run natively on the computing device 500, as a web-based application, or any other format known to those skilled in the art. In some embodiments, application 516 will be a single software program having all of the features and functionality described in the present disclosure. In other embodiments, application 516 may be two or more distinct software programs providing various parts of these features and functionality. For example, application 516 may include one software program for use during the planning phase, and a second software program for use during the treatment phase. In such instances, the various software programs forming part of application 516 may be enabled to communicate with each other and/or import and export various settings and parameters relating to the navigation and treatment and/or the patient to share information. For example, a treatment plan and any of its components generated by one software program during the planning phase may be stored and exported to be used by a second software program during the procedure phase.

Application 516 communicates with a user interface 518 which generates a user interface for presenting visual interactive features to a clinician, for example, on the display 506 and for receiving clinician input, for example, via a user input device. For example, user interface 518 may generate a graphical user interface (GUI) and output the GUI to the display 506 for viewing by a clinician.

The computing device 500 may be linked to the display 110, thus enabling the computing device 500 to control the output on the display 110 along with the output on the display 506. The computing device 500 may control the display 110 to display output which is the same as or similar to the output displayed on the display 506. For example, the output on the display 506 may be mirrored on the display 110. Alternatively, the computing device 500 may control the display 110 to display different output from that displayed on the display 506. For example, the display 110 may be controlled to display guidance images and information during the surgical procedure, while the display 506 is controlled to display other output, such as configuration or status information of an electrosurgical generator 101 as shown in FIG. 1.

Although embodiments have been described in detail with reference to the accompanying drawings for the purpose of illustration and description, it is to be understood that the inventive processes and apparatus are not to be construed as limited thereby. It will be apparent to those of ordinary skill in the art that various modifications to the foregoing embodiments may be made without departing from the scope of the disclosure.

**Claims**

1.  A system for sensing a position of an ultrasound imaging device comprising:

    an ultrasound (US) imaging device (140); and
    a position sensor mount (300) including:

       an elastic body portion (330);
       a first rigid shell (310) disposed on a distal portion of the elastic body portion;
       a second rigid shell (320) disposed on a proximal portion of the elastic body portion; and
       a first adhesive (313) disposed on the first rigid shell; and

    a position sensor mounted on the first rigid shell and configured to sense position information of the US imaging device in a coordinate system, wherein the US imaging device is received by the position sensor mount and circumscribed by the first adhesive.

2.  The system according to claim 1, wherein the first rigid shell includes a first recess and a second recess.

3.  The system according to claim 2, wherein the first recess and the second recess have a same angle with respect to a longitudinal axis of the US imaging device in an opposite direction.

4.  The system according to claim 3, wherein the position sensor includes a first sensor and a second sensor.

5.  The system according to claim 4, wherein the first and second sensors are 5 degrees of freedom sensors.

6.  The system according to claim 4 or 5, wherein the first sensor is positioned on the first recess and the second sensor is positioned on the second recess.

7.  The system according to any preceding claim, wherein the position sensor includes a conductor configured to transmit sensed result.

8.  The system according to claim 7, wherein the elastic body portion includes a recess along a longitudinal axis.

9.  The system according to claim 8, wherein the conductor is positioned on the recess of the elastic body portion.

10. The system according to claim 8, wherein the second rigid shell includes a recess along the longitudinal axis of the US imaging device.

11. The system according to claim 10, wherein the conductor is positioned on the recess of the second rigid shell.

12. The system according to any preceding claim, wherein the coordinate system is based on an electromagnetic field.

13. The system according to claim 12, wherein the position sensor is an electromagnetic sensor.

14. The system according to any preceding claim, wherein the position sensor is located at a first predetermined distance from a distal tip of the US imaging device.

15. The system according to claim 14, wherein the position information of the US imaging device is based on sensed results of the position sensor and the first

**Patentansprüche**

1. System zur Erfassung einer Position einer Ultraschallbildgebungsvorrichtung umfassend:

   eine Ultraschall-(US)-Bildgebungsvorrichtung (140); und
   eine Positionssensorbefestigung (300) aufweisend:

   einen elastischen Körperabschnitt (330);
   eine erste starre Hülle (310), die an einem distalen Abschnitt des elastischen Körperabschnitts angeordnet ist;
   eine zweite starre Hülle (320), die an einem proximalen Abschnitt des elastischen Körperabschnitts angeordnet ist; und
   einen ersten Klebstoff (313), der auf der ersten starren Hülle angeordnet ist; und

   einen Positionssensor, der an der ersten starren Hülle befestigt und konfiguriert ist, um Positionsinformation der US-Bildgebungsvorrichtung in einem Koordinatensystem zu erfassen,
   wobei die US-Bildgebungsvorrichtung von der Positionssensorbefestigung aufgenommen und von dem ersten Klebstoff umschrieben wird.

2. System nach Anspruch 1, wobei die erste starre Hülle eine erste Aussparung und eine zweite Aussparung aufweist.

3. System nach Anspruch 2, wobei die erste Aussparung und die zweite Aussparung einen gleichen Winkel in Bezug auf eine Längsachse der US-Bildgebungsvorrichtung in einer entgegengesetzten Richtung aufweisen.

4. System nach Anspruch 3, wobei der Positionssensor einen ersten Sensor und einen zweiten Sensor aufweist.

5. System nach Anspruch 4, wobei die ersten und zweiten Sensoren 5 Freiheitsgrade-Sensoren sind.

6. System nach Anspruch 4 oder 5, wobei der erste Sensor auf der ersten Aussparung positioniert ist und der zweite Sensor auf der zweiten Aussparung positioniert ist.

7. System nach einem vorstehenden Anspruch, wobei der Positionssensor einen Leiter aufweist, der konfiguriert ist, um ein erfasstes Ergebnis zu übertragen.

8. System nach Anspruch 7, wobei der elastische Körperabschnitt eine Aussparung entlang einer Längsachse aufweist.

9. System nach Anspruch 8, wobei der Leiter auf der Aussparung des elastischen Körperabschnitts positioniert ist.

10. System nach Anspruch 8, wobei die zweite starre Hülle eine Aussparung entlang der Längsachse der US-Bildgebungsvorrichtung aufweist.

11. System nach Anspruch 10, wobei der Leiter auf der Aussparung der zweiten starren Hülle positioniert ist.

12. System nach einem vorstehenden Anspruch, wobei das Koordinatensystem auf einem elektromagnetischen Feld basiert.

13. System nach Anspruch 12, wobei der Positionssensor ein elektromagnetischer Sensor ist.

14. System nach einem vorstehenden Anspruch, wobei der Positionssensor in einem ersten vorbestimmten Abstand von einer distalen Spitze der US-Bildgebungsvorrichtung angeordnet ist.

15. System nach Anspruch 14, wobei die Positionsinformation der US-Bildgebungsvorrichtung auf erfassten Ergebnissen des Positionssensors und dem ersten vorbestimmten Abstand basiert.

**Revendications**

1. Système de détection d'une position d'un dispositif d'imagerie ultrasonore comprenant :

   un dispositif d'imagerie ultrasonore (US) (140) ; et
   un support de capteur de position (300) incluant :

   une partie de corps élastique (330) ;
   une première enveloppe rigide (310) disposée sur une partie distale de la partie de corps élastique ;
   une seconde enveloppe rigide (320) disposée sur une partie proximale de la partie de corps élastique ; et
   un premier adhésif (313) disposé sur la première enveloppe rigide ; et

   un capteur de position monté sur la première enveloppe rigide et configuré pour détecter des informations de position du dispositif d'imagerie US dans un système de coordonnées,
   dans lequel le dispositif d'imagerie US est reçu par le support de capteur de position et limité par le premier adhésif.

2. Système selon la revendication 1, dans lequel la première enveloppe rigide inclut un premier évidement et un second évidement.

3. Système selon la revendication 2, dans lequel le premier évidement et le second évidement présentent un même angle par rapport à un axe longitudinal du dispositif d'imagerie US dans une direction opposée.

4. Système selon la revendication 3, dans lequel le capteur de position inclut un premier capteur et un second capteur.

5. Système selon la revendication 4, dans lequel les premier et second capteurs sont des capteurs à 5 degrés de liberté.

6. Système selon la revendication 4 ou 5, dans lequel le premier capteur est positionné sur le premier évidement et le second capteur est positionné sur le second évidement.

7. Système selon l'une quelconque des revendications précédentes, dans lequel le capteur de position inclut un conducteur configuré pour transmettre un résultat détecté.

8. Système selon la revendication 7, dans lequel la partie de corps élastique inclut un évidement le long d'un axe longitudinal.

9. Système selon la revendication 8, dans lequel le conducteur est positionné sur l'évidement de la partie de corps élastique.

10. Système selon la revendication 8, dans lequel la seconde enveloppe rigide inclut un évidement le long de l'axe longitudinal du dispositif d'imagerie US.

11. Système selon la revendication 10, dans lequel le conducteur est positionné sur l'évidement de la seconde enveloppe rigide.

12. Système selon l'une quelconque des revendications précédentes, dans lequel le système de coordonnées est basé sur un champ électromagnétique.

13. Système selon la revendication 12, dans lequel le capteur de position est un capteur électromagnétique.

14. Système selon l'une quelconque des revendications précédentes, dans lequel le capteur de position est situé à une première distance prédéterminée d'une pointe distale du dispositif d'imagerie US.

15. Système selon la revendication 14, dans lequel les informations de position du dispositif d'imagerie US sont basées sur des résultats détectés du capteur de position et la première distance prédéterminée.

FIG. 1

140

210

## FIG. 2A

141

210

a

b

θ

## FIG. 2B

**FIG. 3**

400

410

420

**FIG. 4A**

**FIG. 4B**

**FIG. 4C**

**FIG. 4D**

**FIG. 4E**

COMPUTING DEVICE
500

PROCESSOR
504

DISPLAY
506

NETWORK
INTERFACE
508

MEMORY
502

CT DATA
214

APPLICATION
516

USER
INTERFACE
518

INPUT DEVICE
510

OUTPUT
MODULE
512

# FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100081920 A1